# EUROPEAN PATENT SPECIFICATION

(11) **EP 2 891 205 B1**
(45) Date of publication and mention of the grant of the patent: **16.11.2016**
(21) Application number: 13759960.1
(22) Date of filing: 29.08.2013
(51) Int. Cl.: H01M 8/06, A61L 2/10, H01M 8/04

(54) **AIRCRAFT GALLEY AND LAVATORY DISINFECTION**
DESINFEKTION FÜR FLUGZEUGBORDKÜCHE UND -TOILETTE
DÉSINFECTION D'OFFICE ET DE TOILETTES D'AÉRONEF

(30) Priority: 29.08.2012 US 201261694312 P; 29.08.2012 US 201261694318 P; 29.08.2012 US 201261694322 P; 29.08.2012 US 201261694307 P; 27.06.2013 US 201313048188
(43) Date of publication of application: 08.07.2015
(60) Divisional application: 16190684.7
(73) Proprietor: MAG Aerospace Industries, LLC, Carson, CA 90746-4012 (US)
(72) Inventor: VANDERVOSSEN, Jos, NL-3702 AP Zeist, Utrecht (NL); BOODAGHIANS, Razmik, B., Glendale, CA 91214 (US); HOOGEVEEN, Andreas, NL-1602GE Enkhuizen (NL); LIBIS, Jean-Paul, F-91570 Bievres (FR); BRUNAUX, Yannick, 59170 Croix (FR)
(74) Representative: Phillips & Leigh
(86) International application number: PCT/US2013/057211
(87) International publication number: WO 2014/036217

(56) References cited:
- WO-A1-2008/010684
- WO-A2-2006/015390
- CN-U- 202 909 018
- US-A- 4 135 269
- US-A1- 2004 056 201
- US-A1- 2009 133 214

## Description

### FIELD OF THE INVENTION

Embodiments of the present invention relate generally to systems and methods for disinfection of aircraft galleys and lavatories and other surfaces. The embodiments may use UV light sources and/or a disinfection unit powered by a fuel cell or other external power source.

### BACKGROUND

Current galley and lavatory cleaning is generally conducted by an airline cleaning company on a frequent basis, and typically during aircraft turn-around times. During this cleaning procedures, chemical disinfection is used to clean exposed surfaces, such as countertops, sinks, trolley doors, and floors. The interior of compartments may also be cleaned on a regular basis, such as ovens, chillers, storage containers, and so forth. During flights, the crew will only clean the galley (or lavatory) area(s) when debris or spillovers are visible. Therefore, additional disinfection is not being carried out during the aircraft flight time.

In the lavatory, the toilets, countertops, cabinet doors, sinks and floors are also cleaned by the cleaning company during aircraft turn-around. Aircraft lavatories provide an environment that is often considered unclean due to the presence of microbiological contamination. There may also be an increased risk for transmissible disease because the standard of hygiene can be very poor in aircraft lavatories, which are used by hundreds of users, without always receiving proper, regular disinfection. Since various pathogens can live for weeks on hard surfaces, various viruses or bacterial infection may spread more easily in aircraft lavatories. Additionally, air traveler immune systems are constantly under assault from lack of circadian rhythms, stress, pesticides and chemicals used in aircraft, foreign environments, and so forth, which can escalate their vulnerability to various pathogens. As the pathogenic landscape continues to morph daily, it is desirable that that health risks associated with contaminated lavatories be addressed. US 2004/056201 (Fink Ronald G et al) relates to a modular, adjustable, easy to maintain, portable food sanitation hood system, comprising a hooded means for subjecting food to sanitizers including UV light, ozone and hydroxyl radicals. The means for subjecting food to the sanitizers includes one or more UV radiation sources and one or more target rods for UV radiation located under a hood. The hood preferably includes an adjustable light curtain to at least partially reduce radiation emitted away from the food. US 4135269 (Marston Laurel L) relates to a mop sterilizer and dryer utilizes a wheel-supported cart having a pair of drainable compartments therein. One of the drainable compartments has an open-mouth portion carrying a pair of squeeze rolls adapted to squeeze the fabric cord-like elements of a mop therebetween. Water captured within such compartment is strained and carried by the cart. The other compartment is totally lined with a reflective material adapted to reflect ultraviolet rays generated by ultraviolet lamp sources disposed within the other compartment. WO 2008/010684 (Hong In-Pyo) relates to a stand type sterilization device using a discharge tube. The stand type sterilization device includes a prop having a cylindrical shape so as to receive a ballast therein, and provided with a plurality of legs installed on the lower end of the circumferential surface thereof in the radial direction, casters attached to the lower surfaces of the legs, and a fastening protrusion formed on the central part of the upper surface thereof; a hollow support rod provided with a screw thread formed on the lower end thereof so as to be screw-connected to the fastening protrusion of the prop and a first connection protrusion formed on the upper end thereof, and having a two-step structure so that the length of the support rod can be adjusted; and an ultraviolet irradiator connected to the upper end of the support rod such that the angle of the ultraviolet irradiator is vertically adjustable. WO 2006/015390 (Perunicic Dragoljub, Perunicic Dragoljub) relates to a cleaning or sterilizing apparatus combined with an UV lamp consisting of an upright compact vacuum cleaner comprising a dust container with a transparent bottom and a suction nozzle incorporated in the bottom and a protective housing which covers the dust container and which incorporates a fan with a centrifugal dust filter and an UV germicidal lamp fixture for destroying micro-organisms on a surface to be cleaned and inside the dust container. US 2009/133214 (Yoo Dong-Hun et al) relates to a nozzle assembly having a UV generation unit and a vacuum cleaner having the same are provided. The nozzle assembly includes a nozzle main body that moves over a cleaning surface, a drum brush unit that is formed on the nozzle main body and includes an extension unit, an ultraviolet (UV) generation unit that is formed on the drum brush unit to be folded or unfolded so that the UV generation unit selectively faces the cleaning surface, and a switch that turns the UV generation unit on or off according to whether the UV generation unit is folded or unfolded.

### BRIEF SUMMARY

The invention is defined by claim 1.

Embodiments of the invention described herein thus provide systems and methods for disinfection of aircraft galleys and lavatories and other surfaces. The embodiments may use UV light sources and/or a disinfection unit powered by a fuel cell or other external power source.

### BRIEF DESCRIPTION OF THE DRAWINGS

FIG. 1 shows a side plan view of a mobile disinfection unit disinfecting an aircraft galley.
FIG. 2 shows a front perspective of one embodiment of a mobile disinfection unit.
FIG. 3 shows a side plan of one embodiment of a mobile disinfection unit.
FIG. 4 shows a front perspective of one embodiment of a mobile disinfection unit.
FIG. 5 shows a side plan view of one embodiment of a mobile disinfection unit.
FIG. 6 shows a use of a fixed disinfection unit in and aircraft lavatory.
FIG. 7 shows a top perspective view of one embodiment of a fixed disinfection unit in an aircraft lavatory.
FIG. 8 shows a top perspective view of one embodiment of a fixed disinfection unit in an aircraft lavatory near the sink area.
FIG. 9 shows a side perspective view of the fixed disinfection unit of FIG. 8 in use.
FIG. 10 shows a side perspective view of one embodiment of a fixed disinfection unit in an aircraft lavatory ceiling.

### DETAILED DESCRIPTION

Embodiments of the present invention provide systems and methods for disinfection of various surfaces, particularly disinfection of aircraft galleys and lavatories. In one embodiment, there is provided a mobile, galley-sized cart that can project a UV light source on a surface to be treated. This embodiment provides a unit that can rolled out, disinfection can be conducted, and then the unit is rolled back into a galley cart storage area. In another embodiment, there is provided a built-in disinfection unit. This built-in unit may be mounted in either one or more the galleys or one or more lavatories. In the lavatory embodiment, the unit may be activated prior to a user entering the lavatory. Whether provided as mobile or built-in units, the disinfection units described herein may be powered by a fuel cell or other external power source. If powered by a fuel cell, the disinfection unit may also use one or more of the fuel cell system by-products, such as heat, water, or oxygen depleted air in order to assist with the disinfection process.

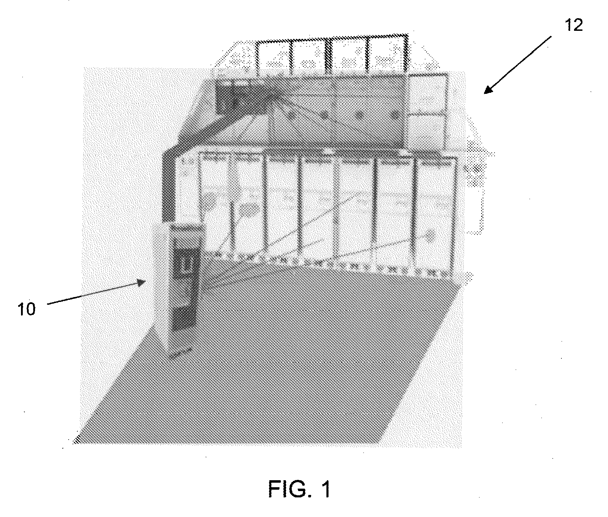

More specifically, Figure 1 shows one embodiment of a mobile disinfection unit 10, disinfecting an aircraft galley area 12. As shown, an aircraft galley 12 has a plurality of surfaces that need to be cleaned--and that can benefit from more frequent cleaning during aircraft flight time. Additionally, being able to disinfect the galley area 12 during flight time could also help decrease the complete "on ground time" of the aircraft, which increases the flying time of the aircraft during its lifetime. This provides a direct benefit to the airliner, because aircraft "on ground time" consumes money rather than makes money. Further, providing a cleaning and disinfection procedure with higher efficiency gives more convenience to the cleaning crew and the airliner.

The mobile disinfection unit 10 may be provided as a high efficiency mobile cleaning and disinfection unit that has one or more ultraviolet (UV) light sources 14 (either with or without photo-catalytic oxidation) that can be used to treat the surfaces of the galley. By placing the mobile disinfection unit 10 in front of the galley 12, the UV light can be used to treat the exposed surfaces. Additionally and/or alternatively, the doors of the various galley units (such as ovens, chillers, trash compactors, containers, and any other miscellaneous and trolley compartments) may be opened, which allows the internal surfaces of these components to be exposed to the UV light as well.

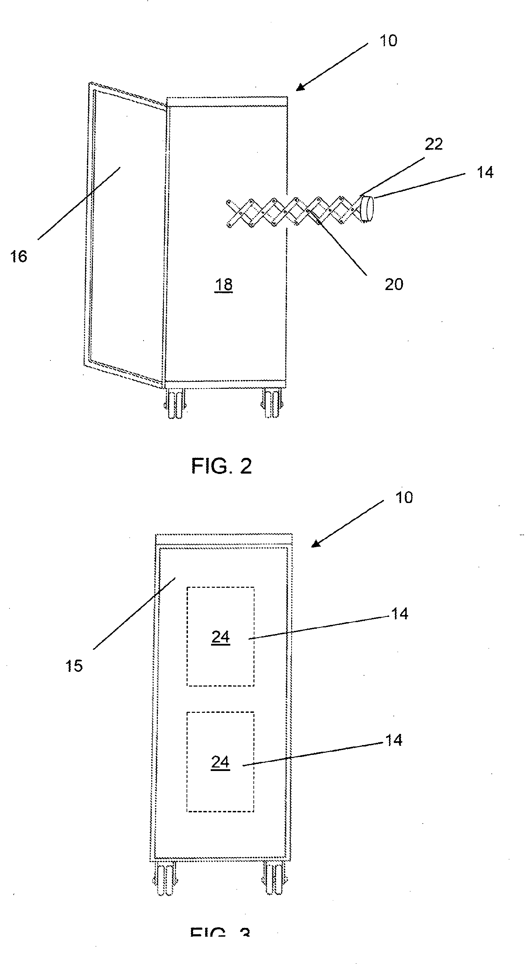

In one embodiment, the one or more UV light source(s) 14 may be housed inside the unit 10 on a retractable mast that is fixed in the unit but that can rotate to adjust the direction of the UV light source 14. In one embodiment, as shown in Figure 2, the unit may have the shape of a typical galley trolley, with a door 16 that opens to expose an interior 18. A retractable mast 20 may be pulled out of the unit 10, the mast 20 having a rotatable head 22 that supports the UV light source 14. One or more UV light sources 14 may be positioned on a mast and one or more masts 20 may be positioned inside the unit 10. The retraction and rotation of the mast 20 is adjustable and may either be electrically powered or manually powered.

Additionally or alternatively, the unit 10 may have a side panel 15 with one or more separate UV panels 24 with one or more mounted UV light source(s) 14 positioned thereon, as shown in Figure 3. The mounted UV light sources 26 may operate together or on a sequence in order to disinfect all parts of the galley (or lavatory or other aircraft area to be disinfected) independent of the height of the unit. In this embodiment, the unit 10 may be pulled out from its stowage position, and the mounted UV light source(s) 14 is/are activated. The mast 20 may or may not be provided and/or activated as well. As described below, the unit 10 may have certain independent drive features, and if activated, the unit 10 may be allowed to rotate or move around an axis so that all surfaces is a 360° radius may be treated by the mounted UV light source(s) 26.

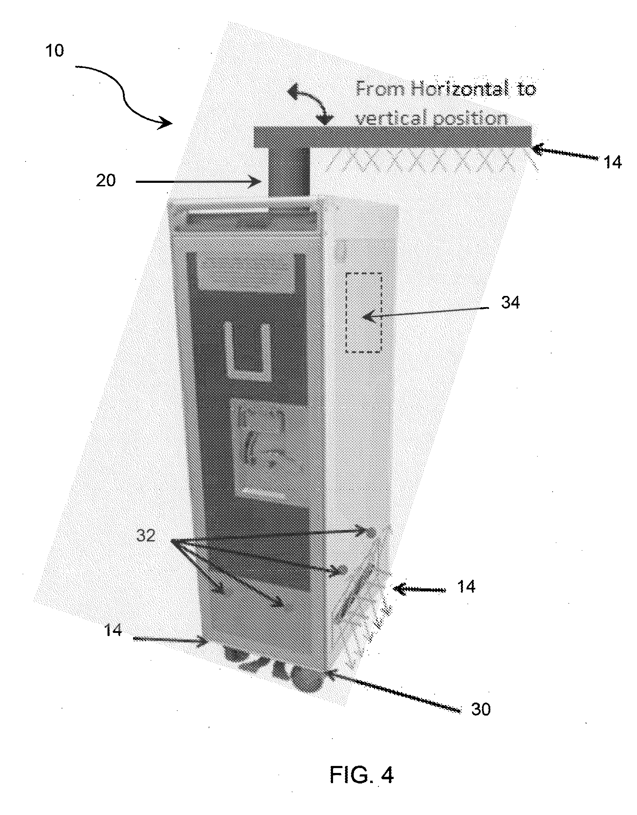

In the embodiment shown in Figure 4, the unit 10 may have a mast 20 that extends from the upper portion of the unit 10, as is shown as extending from the top of unit. The mast may be height adjustable and rotatable, so that all areas of the surface to be disinfected may be treated. The mast 20 may also move from horizontal to vertical so that it can treat area of varying heights and distances. This may be done via a hinge, via a removable wand (such that the mast is removable from the base unit 10 and can be passed over a surface), or via any other appropriate system.

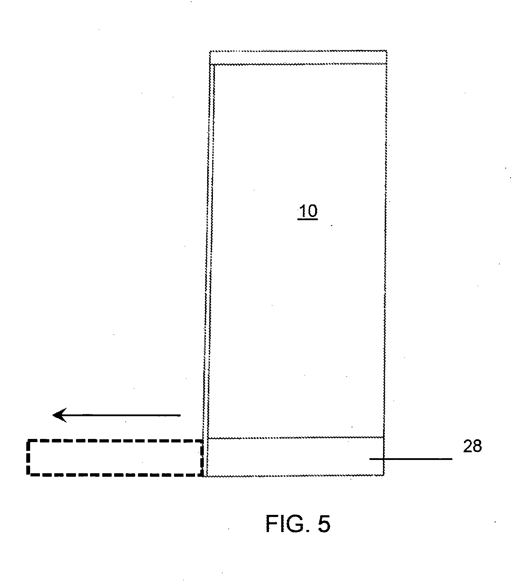

In a further embodiment as shown in Figure 5, the mobile disinfection unit 10 may have one or more UV light sources positioned on a lower portion of the unit 10 or on a bottom panel 28 in order to disinfect the floor underneath the unit 10. The position of the UV light source(s) on the bottom of the unit is generally structured in a way that allows the light source to adjust by stretching out in order to expose of more floor surface, if needed. For example, one or more slidable panels with UV light sources(s) positioned thereon may be provided on the bottom of the unit that can slide out and allow more UV light source treatment.

In any of the embodiments described herein, it is possible to design the unit 10 so that it minimizes any UV light escaping from the housing, in order to prevent harm to the operators during use of the unit 10. There may also be focused shields or cones positioned on or near the UV light source to direct the light as desired and to prevent the light from reaching into undesired areas (where unprotected people may be located). This mode of application can prevent any reflected light from the galley reaching other surfaces, reflecting therefrom, and/or from directly shining into an area where persons may be located. It is also possible for any of the embodiments described herein to have a remote operating feature, which allows the mobile unit to be operated (either to drive to unit to the appropriate location and/or for activation of the UV light once the unit has been positioned as desired). This remote operation is also intended to prevent harm to operators or those in surrounding areas (such as the crew, cleaning staff, passengers, and so forth).

In a further embodiment, the mobile disinfection unit 10 may also have a secondary cleaning system 30, such as a vacuum. This may be provided as a built-in capability to remove / collect any debris from the floor of the trolley bay area, either manually or automatically by means of a vacuum cleaner. This system 30 may be positioned at a lower level of the unit 10, such that movement of the unit 10 functions to vacuum up debris. Alternatively, system 30 may be an attachment that is positioned on the side of the unit, with a removable hose that can be attached for vacuuming purposes or removed for storage purposes.

The size of and shape of the mobile disinfection unit 10 will generally be compatible with current trolley and galley insert standards. This allows the unit 10 to be removed for use, but stowed in the galley during taxi, take-off, and landing, and any other times when the unit 10 is not in use. Alternatively, the mobile unit can be the size of a standard aircraft storage container such that it can be removed for use, or used when in position in the aircraft cabinet areas, and returned or shut down after use.

The mobile disinfection unit 10 can be operated on the ground and / or on-board during flight. If used in flight, the area which is intended to be cleaned and/or disinfected should be closed/confined by means of a curtain, screen or door to block the UV light. There may also be a time delay function provided, which allows time for the curtains, screens, or door to be drawn, prior to the UV light source activation, in order to prevent eye damage to anyone in the vicinity. If someone is detected in the area of UV radiation, a system may be provided so that the light is automatically switched off.

The mobile disinfection unit 10 may have a drive assist feature, to minimize the effort needed by the user. In addition, proximity sensors 32 may be included to assist with the navigation and prevent the unit 10 from hitting or bumping into obstacles by being integrated with a steering mechanism. In one embodiment, the mobile disinfection unit may have a smart sensor that detects obstacles in its way. This can allow the unit 10 to function like a robotic vacuuming/disinfecting unit, moving its way through the galley. This is particularly useful for on ground cleaning, when passengers and crew are not on-board.

The mobile disinfection unit may be self-powered by a battery or a fuel cell or any external power source. The powering feature of the unit is shown schematically as 34 in Figure 4. If the unit 10 is powered via a fuel cell, one advantage is that the byproducts of the fuel cell system may be used to enhance the disinfection features, as described in more detail below. Unit 10 may have an electrical control unit that automatically stops the operation of unit 10 after the cleaning process is completed and/or after a certain amount of treatment time has elapsed. The unit 10 may also be provided with a redundant auto-stop device with a delayed start sequence, and or provided by remote controlled operation.

In addition to disinfecting the galley, the unit 10 may be rolled into the lavatory for treatment. (As discussed above, the fixed unit may also feature a secondary cleaning system 30 with the built-in capability to remove / collect any debris, discarded tissues, and spillovers in the lavatory. The vacuum may be able to vacuum debris as well as excess liquid or spills. If the unit 10 and/or the secondary cleaning system 30 is used manually, a trained person with appropriate personal protection should be employed. Alternatively, the unit 10 / secondary cleaning system 30 may be activated automatically by a vacuum system provided in the unit.) The unit 10 may be sized similarly to the galley trolley cart unit of Figures 1-5, or it may be sized for storage elsewhere, such as in the lavatory, under the sink, under the toilet, or positioned inside a sing away mirror compartment, or elsewhere.

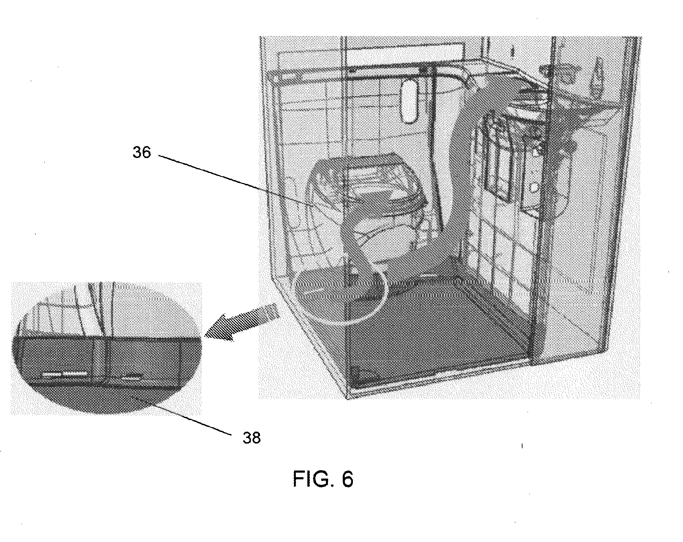

Another embodiment provides a fixed disinfection unit that is mounted in the lavatory for space considerations and for easy, targeted disinfection. One example of a small fixed disinfection unit 38 is shown in Figure 6, which illustrates the unit at a lower portion of a lavatory toilet 38. This unit 38 includes a UV light source that may be covered by a window or other protective liner/covering. When the unit is activated, the UV light is allowed to surround the areas on or near the toilet for disinfection, such as the floor area, the toilet lines and vent line tubing, the toilet seat, the toilet shroud, and other surrounding areas. As illustrated by the arrows in this figure, the UV light may be sent upward to disinfect surfaces. For example, the light may disinfect the toilet shroud, which can harbor bacteria. It can also disinfect the sink and wall areas as well. It is proposed that the unit 38 may be used to treat the surfaces of the lavatory, the air, and any other parts of a lavatory such as the toilet, sink, or countertop, by positioning the mobile UV light unit inside the lavatory.

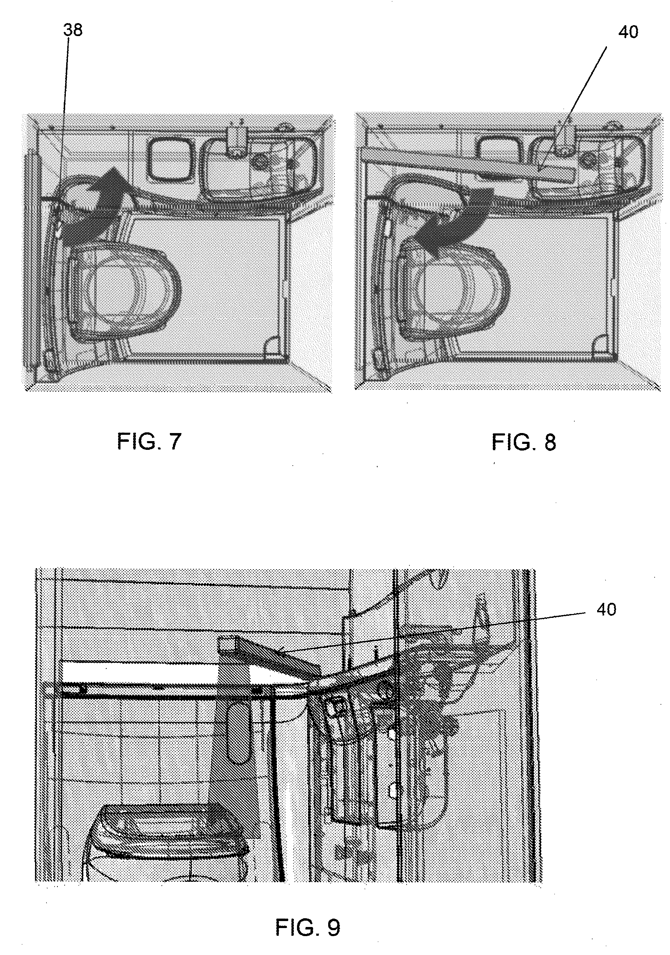

Figure 7 illustrates an alternate position for the unit 38 near the back wall, above the toilet. Although not shown, it is also possible to provide one or more fixed units 38 positioned behind the sink or anywhere in the lavatory as desired.

Figures 8 and 9 illustrate a swing arm unit 40 that may be mounted on or near a sink countertop area and swung as desired to the disinfection area. Figure 8 shows the arm unit 40 in a stowed position, and Figure 9 shows the arm in a treatment position. This may be a manual movement, such that a passenger or crew member may move the arm unit 40 and depress a button for immediate surface disinfection. Additionally or alternatively, the swing arm unit 40 may move upon a command that is issued from outside the lavatory when the door is closed/locked. One or more of the features/options may be used in combination.

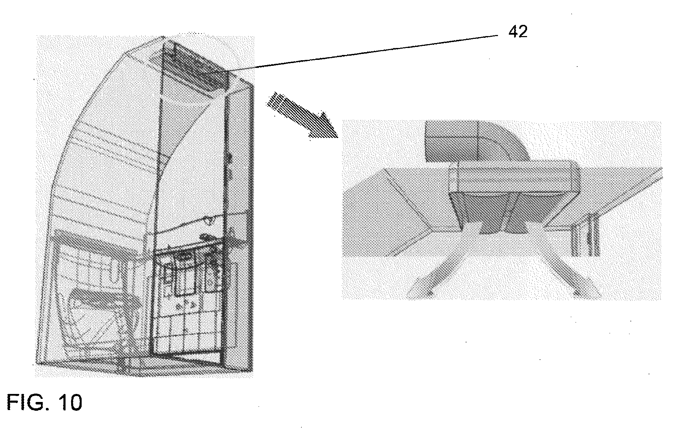

Figure 10 shows one embodiment of a fixed disinfection unit 42 in an aircraft lavatory ceiling. In this embodiment, there may be a cover or flap that closes over the unit when not in use, and when activated the cover opens to allow UV light to penetrate the lavatory space. This may be used to clean surfaces, as well as the air in the lavatory in order to disinfect the air and remove odors.

In any of the embodiments described, the disinfection module may be a built-in and/or an add-on system that is programmed to operate automatically prior to and/or after the lavatory is used. The user may be offered an option to activate the disinfection process prior entering the lavatory. Once the option is activated, the lavatory door locks automatically, preventing the user entering the lavatory until the disinfection process is complete. The disinfection process will be of short duration, e.g., a matter of seconds. In case of an intrusion, the process will be aborted in order to protect the intruder's eyes from the UV light. Any debris, discarded tissues and spillovers may be removed automatically via an optional vacuum system that may be provided. For example, toilet droplets and sink droplets can be unsanitary, and although the UV light cannot remove them, it can help disinfect them. Providing an optional vacuum system may also allow removal of any moisture in the offending area. In order to disinfect the toilet ring and the lid, it is proposed that the ring and lid may be made of UV light transmitting material, such as a transparent material that is compatible with UV disinfection light, so that the light can fully treat these surfaces even if the toilet lid is closed.

The UV light source module may comprise one or more lamps that are strategically positioned inside the lavatory to increase the exposure of the surfaces to UV light, in particular, the door handle and any other door opening/closing/locking mechanism. The lamps can be operated individually, together, or in a sequence.

In one embodiment, in addition to the use of UV light for sanitation, hot water, hot air, or steam may be used for cleaning surfaces inside that lavatory and in the galley. In some embodiments, the heated water and/or air may be provided as one of the by-product outputs of a fuel call reaction. The heated air may also be used for drying wet surfaces in the lavatory, galley, and/or aircraft cabin interior, regardless of prior UV treatment. Hot oxygen depleted air (ODA) and/or water products (e.g. steam) from the fuel cell may be used alone or combined with UV treatment to clean equipment or surfaces, such as may be included in lavatories, galleys, sinks, and the aircraft cabin. Additionally, as a substitute or complement to UV treatment, the ODA can be treated by initially removing the moisture from the ODA and raising its temperature, if desired.

As discussed, the UV treatment can also be used alone (i.e., without supplementation by fuel cell products) to sanitize areas within the aircraft. There are embodiments envisioned that do not use the formal disinfection unit as well. For example, when the lavatory is not in use, a sliding UV treatment probe may be provided inside a cabinet and removed to be deployed over the toilet seat or counter surface, in order to expose the surface to germicidal light for sanitation. Such a probe may also be utilized on food preparation surfaces in galleys or in other areas within the aircraft. Alternatively, larger banks of germicidal light sources can be utilized in place of probes in order to treat an entire area, such as a lavatory, when it is not occupied by a passenger.

## Claims

1. A mobile disinfection unit (10), comprising
(a) an aircraft trolley-sized body,
(b) one or more UV light sources (14) associated with the trolley body and configured to disinfect one or more aircraft surfaces, the one or more UV light sources (14) are positioned on a movable mast (20);
**characterised in that** the mast (20) can be pulled out of the body, and the mast (20) having a rotatable head (22) that supports the one or more UV light sources (14).

2. The unit (10) of either of claims 1 or 2, wherein the movable mast (20) is configured to (i) extend and retract, (ii) move vertically and horizontally, or combinations thereof.

3. The unit (10) of any of the preceding claims, wherein the one or more UV light sources (14) are positioned on a side panel of the trolley.

4. The unit (10) of any of the preceding claims, wherein the one or more UV light sources (14) are positioned below the trolley.

5. The unit (10) of any of the preceding claims, further comprising a vacuum system (30) associated with the trolley body.

6. The unit (10) of any of the preceding claims, further comprising one or more proximity sensors (32) associated with the trolley body.

7. The unit (10) of any of the preceding claims, wherein the unit comprises a curtain or screen or door to prevent UV light from escaping when unintended.

8. The unit (10) of any of the preceding claims, wherein the unit comprises a system to automatically switch off the UV light sources (14) when someone is detected in the area of the UV radiation.

9. The unit (10) of any of the preceding claims, wherein the one or more UV light sources (14) are powered by a fuel cell positioned in the trolley body.

10. A method of disinfecting aircraft surfaces by use of the mobile disinfection unit according to claim 1 comprising the step of:
i) using one or more by-products of a fuel cell of a mobile disinfection unit (10) as defined in claim 9.

11. The method of claim 10, wherein the one or more by-products of the fuel cell include
a) water,
b) heat, and
c) oxygen depleted air.

## Patentansprüche

1. Eine mobile Desinfektionseinheit (10), umfassend
(a) einen Wagenaufbau mit den Abmessungen eines Flugzeug-Servierwagens,
(b) eine oder mehrere UV-Lichtquellen (14), die mit dem Wagenaufbau verbunden sind und konfiguriert sind, eine oder mehrere Oberflächen eines Flugzeugs zu desinfizieren, wobei die eine oder mehreren UV-Lichtquellen (14) an einem beweglichen Mast (20) angeordnet sind;
**dadurch gekennzeichnet, dass** der Mast (20) aus dem Aufbau ausgefahren werden kann, und der Mast (20) einen drehbaren Kopf (22) aufweist, der die eine oder die mehreren Lichtquellen (14) trägt.

2. Die Einheit (10) gemäß Anspruch 1, wobei der bewegliche Mast (20) konfiguriert ist, (i) auszufahren und einzufahren, (ii) sich vertikal und horizontal zu bewegen, oder Kombinationen davon.

3. Die Einheit (10) gemäß irgendeinem der vorhergehenden Ansprüche, wobei die eine oder mehreren UV-Lichtquellen (14) an einer Seitenwand des Wagens positioniert sind.

4. Die Einheit (10) gemäß irgendeinem der vorhergehenden Ansprüche, wobei die eine oder mehreren UV-Lichtquellen (14) unter dem Wagen positioniert sind.

5. Die Einheit (10) gemäß irgendeinem der vorhergehenden Ansprüche, ferner umfassend ein Vakuumsystem (30), das mit dem Wagenaufbau verbunden ist.

6. Die Einheit (10) gemäß irgendeinem der vorhergehenden Ansprüche, weiterhin umfassend einen oder mehrere Annäherungssensoren (32), die mit dem Wagenaufbau verbunden sind.

7. Die Einheit (10) gemäß irgendeinem der vorhergehenden Ansprüche, wobei die Einheit einen Vorhang oder eine Abschirmung oder eine Tür aufweist, um ein unbeabsichtigtes Entweichen des UV-Lichtes zu verhindern.

8. Die Einheit (10) gemäß irgendeinem der vorhergehenden Ansprüche, wobei die Einheit ein System aufweist, um die UV-Lichtquellen (14) automatisch abzuschalten, wenn irgendjemand im Bereich der UV-Strahlung detektiert wird.

9. Die Einheit (10) gemäß irgendeinem der vorhergehenden Ansprüche, wobei die eine oder mehreren UV-Lichtquellen (14) durch eine in dem Wagenaufbau angeordnete Brennstoffzelle mit Energie versorgt werden.

10. Ein Verfahren zur Desinfektion von Oberflächen in einem Flugzeug unter Verwendung der mobilen Desinfektionseinheit gemäß Anspruch 1 enthaltend die Schritte:
i) Verwenden von einem oder mehreren Nebenprodukten einer Brennstoffzelle einer mobilen Desinfektionseinheit (10) wie in Anspruch 9 definiert.

11. Das Verfahren gemäß Anspruch 10, wobei das eine oder die mehreren Nebenprodukte der Brennstoffzelle umfassen:
a) Wasser,
b) Wärme und
c) Sauerstoff-abgereicherte Luft.

## Revendications

1. Unité de désinfection mobile (10), comprenant
(a) un corps en forme d'un chariot d'aéronef,
(b) une ou plusieurs sources de lumière UV (14) associées au corps de chariot et configurées pour désinfecter une ou plusieurs surfaces d'aéronef, les une ou plusieurs sources de lumière UV (14) étant positionnées sur un mât mobile (20) ;
**caractérisée en ce que** le mât (20) peut être tiré vers l'extérieur du corps, et le mât (20) ayant une tête rotative (22) qui supporte les une ou plusieurs sources de lumière UV (14).

2. Unité (10) selon la revendication 1, dans laquelle le mât mobile (20) est configuré pour (i) s'étendre et se rétracter, (ii) se déplacer verticalement et horizontalement, ou l'une de leurs combinaisons.

3. Unité (10) selon l'une quelconque des revendications précédentes, dans laquelle les une ou plusieurs sources de lumière UV (14) sont positionnées sur un panneau latéral du chariot.

4. Unité (10) selon l'une quelconque des revendications précédentes, dans laquelle les une ou plusieurs sources de lumière UV (14) sont positionnées en dessous du chariot.

5. Unité (10) selon l'une quelconque des revendications précédentes, comprenant en outre un système de vide (30) associé au corps de chariot.

6. Unité (10) selon l'une quelconque des revendications précédentes, comprenant en outre un ou plusieurs capteurs de proximité (32) associés au corps de chariot.

7. Unité (10) selon l'une quelconque des revendications précédentes, dans laquelle l'unité comprend un rideau ou un écran ou une porte pour empêcher la lumière UV de s'échapper lorsque cela n'est pas souhaité.

8. Unité (10) selon l'une quelconque des revendications précédentes, dans laquelle l'unité comprend un système pour éteindre automatiquement les sources de lumière UV (14), lorsqu'une personne est détectée dans la zone de rayonnement UV.

9. Unité (10) selon l'une quelconque des revendications précédentes, dans laquelle les une ou plusieurs sources de lumière UV (14) sont alimentées par une pile à combustible positionnée dans le corps de chariot.

10. Procédé de désinfection de surfaces d'aéronef par l'utilisation de l'unité de désinfection mobile selon la revendication 1, comprenant l'étape :
i) d'utilisation d'un ou de plusieurs sous-produits d'une pile à combustible d'une unité de désinfection mobile (10) telle que définie dans la revendication 9.

11. Procédé selon la revendication 10, dans lequel les un ou plusieurs sous-produits de la pile à combustible comprennent
a) de l'eau,
b) de la chaleur, et
c) de l'air appauvri en oxygène.
